# EUROPÄISCHE PATENTSCHRIFT

(11) **EP 3 171 829 B1**
(45) Veröffentlichungstag und Bekanntmachung des Hinweises auf die Patenterteilung: **04.12.2024**
(21) Anmeldenummer: 15744102.3
(22) Anmeldetag: 09.06.2015
(51) Int. Cl.: A61F 5/01, A61F 5/058

(54) **VORRICHTUNG ZUR LINDERUNG DER BESCHWERDEN EINER DAUMENSATTELGELENKSERKRANKUNG AUS DEM DEGENERATIVEN, ENTZÜNDLICHEN UND RHEUMATISCHEN FORMENKREIS.**
DEVICE FOR ALLEVIATING THE COMPLAINTS OF A TRAPEZIOMETACARPAL JOINT DISEASE FROM THE DEGENERATIVE, INFLAMMATORY, AND RHEUMATIC SPECTRUM
DISPOSITIF POUR SOULAGER LES DOULEURS PROVOQUÉES PAR UNE AFFECTION DE L'ARTICULATION CARPO-MÉTACARPIENNE DU POUCE DE TYPE DÉGÉNÉRATIF, INFLAMMATOIRE ET RHUMATISMAL

(30) Priorität: 22.07.2014 DE 202014103362 U
(43) Veröffentlichungstag der Anmeldung: 31.05.2017
(73) Patentinhaber: Meyer, Christine, 49751 Sögel (DE)
(72) Erfinder: Meyer, Christine, 49751 Sögel (DE)
(74) Vertreter: Gramm, Lins & Partner Patent- und Rechtsanwälte PartGmbB
(86) Internationale Anmeldenummer: PCT/DE2015/100229
(87) Internationale Veröffentlichungsnummer: WO 2016/011999

(56) Entgegenhaltungen:
- WO-A2-2007/066367
- DE-A1- 19 511 116
- DE-U1- 202006 011 664
- US-A- 5 771 901

## Beschreibung

Die Erfindung betrifft eine Vorrichtung zur Linderung der Beschwerden einer Daumensattelgelenkserkrankung aus dem degenerativen, entzündlichen und rheumatischen Formenkreis.

Insbesondere bei der Rhizarthrose handelt es sich um eine Erkrankung des Daumensattelgelenks, infolge dessen es zu unspezifischen Abnutzungserscheinungen einhergehend mit einer Verringerung der Gleitschicht des Knorpelgelenks bis hin zu dessen vollständiger Auflösung kommen kann, verbunden mit zunehmend schmerzhafter Entzündung und Druckempfindlichkeit. Des Weiteren wird der Kapsel-Band-Apparat sekundär geschädigt und geschwächt.

Bisher bekannte Behandlungsmethoden stellen das Gelenk ruhig, wodurch als schmerzhaft empfundene Bewegungen und Belastungen mehr oder weniger effektiv unterbunden werden.

In DE 3006 362 A1 wird eine Erfindung aus zwei offenen, am Unterarm befestigten Stegen beschrieben, bei der unter anderem der Daumen in einer zum Schienenende verlaufenden Hülse so eingeführt ist, dass die gesamte Hand einschließlich des Handgelenks bewegungsunfähig arretiert ist.

DE 3519493 A1 offenbart eine Vorrichtung zum Ruhigstellen des Daumen-Sattelgelenks, bei der der Daumen und die Hand zumindest teilweise von einem festen Formkörper umschlossen fixiert sind. Durch die anatomische Ausformung der Schiene verbleibt die Hand in ihrer natürlichen Lage und bewirkt so die Ruhigstellung des Daumen-Sattelgelenks als auch des Daumen-Grundgelenks.

Auch in WO 2011/110420 A1 wird eine Vorrichtung beansprucht, deren Aufgabe eine spezifische und vollständige Immobilisierung des Daumen-Sattelgelenkes erreicht und so die schmerzhafte Bewegung der von der Arthrose beeinträchtigten Gelenke sicher unterbindet.

Die DE 20 2006 011 664 U1 betrifft eine Mittelhand-Daumenorthese zur Rückstellung der Mittelhand mit einer an Teilbereichen der Hand anlegbaren elastischen Schiene und mindestens einem Verschlusselement, wobei die Schiene in Längserstreckung offen und insgesamt symmetrisch ausgebildet ist und ein Verschlusselement im daumenseitigen Bereich und mindestens ein Verschlusselement im handgelenkseitigen Bereich angeordnet ist. Die Schiene kann aus Kunststoff ausgebildet sein.

Die DE 195 11 116 A1 betrifft eine Daumenorthese aus einem formstabilen und einem Durchgang zum Durchstecken des Daumens aufweisenden Formkörper, der bei angelegter Orthese den Daumen über einen Teil seiner Länge umgibt und an der Hand befestigbar ist. Der Formkörper ist als geschlossener Ring ausgebildet, der distal den Bereich zwischen Daumen und Mittelhand und proximal den Daumen im Bereich des Sattelgelenkes umgibt. Der Ring stützt den Daumenballen volarseitig flächig ab. Der Ring kann thermoplastischem Kunststoff bestehen.

Die WO 2007/066367 A2 stellt den nächstliegenden Stand der Technik dar und betrifft eine Daumenorthese mit einer steifen Abstützung aus einem Metal in einer im Wesentlichen Y-Form, die zumindest zwei Seitenabschnitte und einen zentralen, langgestreckten Abschnitt aufweist. Die beiden Seitenabschnitte umfassen das Handgelenk, der langgestreckte Abschnitt erstreckt sich bis zur Spitze des Daumens und weist eine langgestreckte und gebogene Form korrespondierend zu der anatomischen Form der Hand auf.

Alle Erfindungen haben den Nachteil, dass durch die Ruhigstellung des Gelenks der Kapsel-Band-Muskelapparat in kurzer Zeit erschlafft und infolge dessen eine fortschreitende Einschränkung in der Beweglichkeit einhergeht mit einer Verschlimmerung der Schmerzsymptome.

Des weiteren ist die gewünschte Ruhigstellung nur dann erreichbar, wenn die mitunter auch plastisch verformbare und damit anatomisch angepasste Schiene die zu arretierenden Körperteile fest umschließt. Dadurch wird die Blutzirkulation beeinträchtigt und infolge punktuell wirkenden Drucks werden Empfindlichkeiten und Scheuerstellen hervorgerufen.

Das Ausführen auch nur leichter körperlicher Betätigung wird infolge der Ruhigstellung nahezu komplett unterbunden und der aus Daumen und Mittelfinger bildbare Pinzettengriff deutlich erschwert oder gar unmöglich.

DE 20 2012 009 952 U1 weicht von den vorerwähnten Prinzipien ab und stellt das Daumensattelgelenk nicht nur ruhig, sondern spreizt dieses durch eine in die anatomisch angepasste Orthese eingeklebte flügelförmige Pelotte aus einem nachgiebig gepolsterten Material. Ein Druck auf die Daumenspitze, wie er insbesondere beim Pinzettengriff wirkt, wird so über die anatomisch ausgeformte Orthese auf die Mittelhand abgeleitet, was gleichsam den Druck auf die Pelotte erhöht und die aufeinander reibenden Gelenkschalen zunehmend expandiert.

Dies birgt den Nachteil, dass das Daumensattelgelenk fest umschlossen in einer Schiene ruht. Durch den gegen die Schiene ausgelösten Druck, der durch die eingeklebte Pelotte noch erhöht wird, kann es beim Tragen der Vorrichtung zu punktuellen Belastungen bis hin zu Druckstellen kommen. Das Anpassen der Orthese bereitet darüber hinaus nicht unerhebliche Schwierigkeiten, weil die Pelotte nur exakt ausgerichtet das Auseinanderdrücken des Gelenkes bewirkt, andernfalls den Schmerz der Grunderkrankung sogar verstärkt.

Ziel der Erfindung ist es, eine Vorrichtung bereitzustellen, durch die das arthrotisch veränderte Daumensattelgelenk dynamisch entlastet wird, verbunden mit dem Vorteil, dass der Kapsel-Band-Muskelapparat durch ständige Zug- und Entspannung in seiner natürlichen Bewegung gefordert ist und deshalb nicht erschlafft.

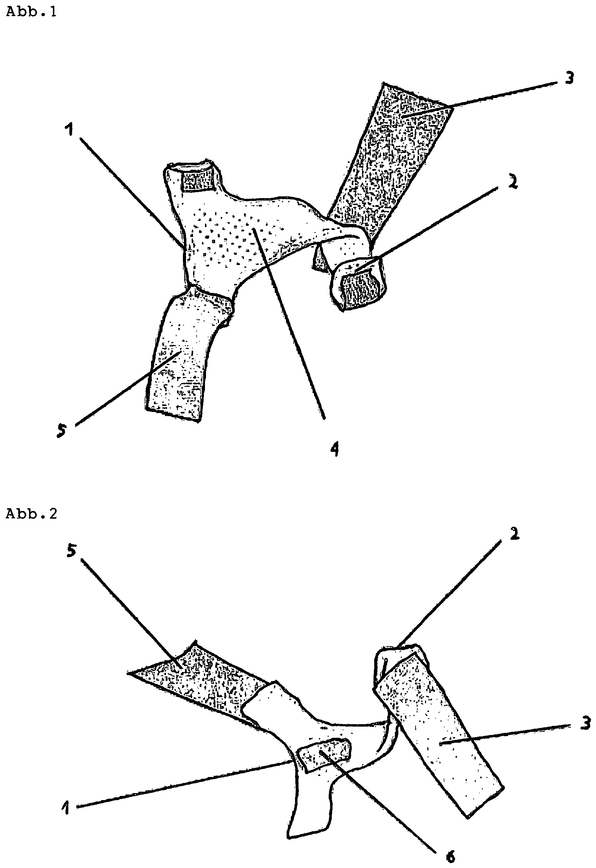

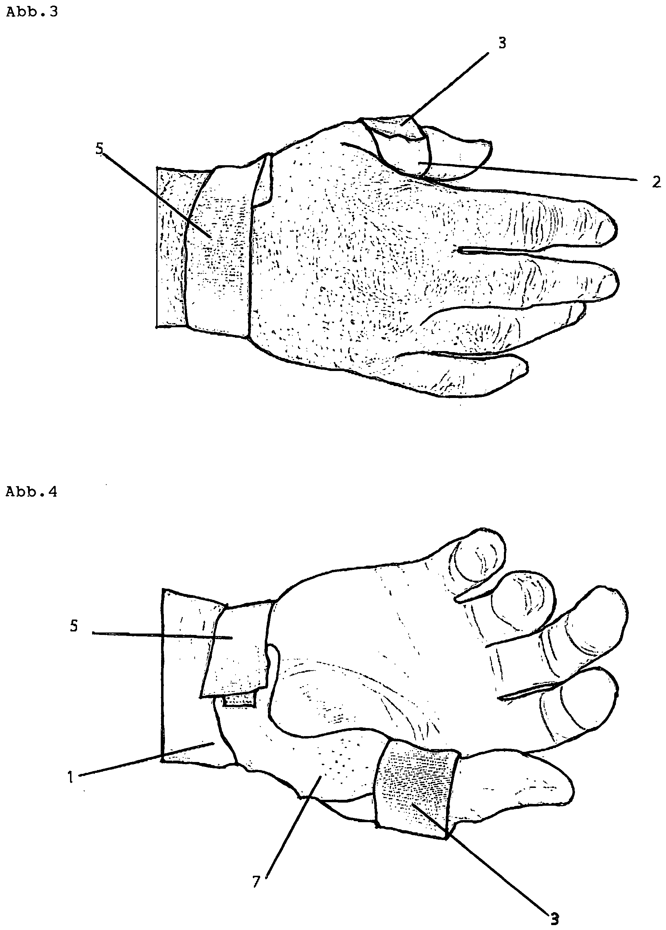

Die Fig. 1 u. Fig. 2 zeigen die Vorrichtung, Fig. 3 u. Fig. 4 zeigen die Vorrichtung im angelegten Zustand.

Die Erfindung besteht aus einer Schiene (Fig. 1 -Fig. 4), lösbar fixierbar (5) am Handgelenk des Patienten einerseits und unterhalb des ersten Daumengelenks der erkrankten Hand andererseits (3). Sie besteht aus einem erstarkten thermoplastischen Kunststoff, der das Handgelenk teilweise umschließt (1), am Handballen (7) passgenau anliegt und mit einer korkenzieherförmigen Linkswindung unterhalb des ersten Daumengelenks abschließt (2).

In ihrer Grundstellung ist die Schiene so ausgebildet, dass sie den Daumen in seiner natürlichen Ruheposition aufnimmt.

Wird nun der Daumen auf den Mittelfinger hin zum Pinzettengriff geführt, spannt der unterhalb des ersten Daumengelenks fixierte Daumen (2) infolge seiner radialen Bewegung die Schiene (4) gegen die am Handgelenk befestigte Halterung (1) und hebt dadurch das Daumensattelgelenk minimal aus seinem angestammten Sitz, wodurch ein Spalt zwischen den ineinandergreifenden Gelenkköpfen entsteht, so dass diese infolge der Bewegung weniger stark bis gar nicht aufeinander reiben, was mit einer spürbaren Verringerung der Schmerzen einhergeht.

Vergleichbar ist die Wirkung beim Beugen des ersten Daumenglieds: Durch die als Widerlager gegen das Handgelenk ausgebildete Schiene hebelt das umschlossene Daumengelenk das Daumensattelgelenk minimal aus der Gelenkmulde und führt so zu einer Druckentlastung der aufeinander stehenden Gelenkknochen.

Durch den dynamischen Prozess der angepassten Entlastung wird der übrige Kapsel-Band-Muskelapparat stetig beansprucht und gefördert. Der weitere Vorteil der Erfindung besteht darin, dass durch den geringen Materialaufwand das Tragegefühl insbesondere durch die geringe Abdeckung der Haut als angenehm und nicht störend empfunden wird.

In bevorzugter Ausgestaltung ist in die Schiene unterhalb des Daumenstattelgrundgelenks (6) eine Pelotte eingearbeitet.

In weiterer bevorzugter Ausgestaltung besteht die Schiene aus gelochtem Kunststoff.

## Patentansprüche

1. Orthese aus Kunststoff, mit einer anatomisch angepassten Schiene (4), wobei die Schiene (4) lösbar fixierbar an einem rechten Handgelenk und proximal eines Daumensattelgelenks der erkrankten Hand ansetzt, **dadurch gekennzeichnet, dass**
die Schiene (4) von radial nach ulnar und von dorsal nach volar über den Handballen (7) mit einer korkenzieherförmigen Linkswindung in distale Richtung zum ersten Daumengelenk geführt unterhalb des ersten Daumengelenkes abschließt.

2. Orthese nach Anspruch 1, **dadurch gekennzeichnet, dass** in die Schiene (4) eine Pelotte zur Anlage proximal des Daumensattelgelenks eingearbeitet ist.

3. Orthese nach Anspruch 1 oder 2, **dadurch gekennzeichnet, dass** die Orthese aus einem gelochten Kunststoff bildbar ist.

4. Orthese nach einem der voranstehenden Ansprüche, **dadurch gekennzeichnet, dass** die Schiene (4) aus einem thermoplastischen Kunststoff besteht.

5. Orthese nach einem der voranstehenden Ansprüche, **dadurch gekennzeichnet, dass** die Schiene (4) das Handgelenk teilweise umschließend ausgebildet ist.

6. Orthese nach einem der voranstehenden Ansprüche, **dadurch gekennzeichnet, dass** die Schiene (4) den Daumen (2) in seiner natürlichen Ruheposition aufnehmend geformt ist.

7. Orthese nach einem der voranstehenden Ansprüche, **dadurch gekennzeichnet, dass** die Schiene (4) das Daumengelenk umschließend und als Widerlager gegen das Handgelenk ausgebildet ist und im angelegten Zustand das Daumensattelgelenk aus der Gelenkmulde hebelt.

## Claims

1. Orthosis made of plastic, with an anatomically adapted splint (4), the splint (4) being detachably attachable to a right wrist and proximal to a thumb saddle joint of the diseased hand, **characterised in that** the splint (4) terminates below the first thumb joint, guided from radial to ulnar and from dorsal to volar over the ball of the hand (7) with a corkscrew-shaped left-hand turn in the distal direction towards the first thumb joint.

2. Orthosis according to claim 1, **characterised in that** a padding is incorporated into the splint (4) for contact proximal to the thumb saddle joint.

3. Orthosis according to claim 1 or 2, **characterised in that** the orthosis can be formed from a perforated plastic.

4. Orthosis according to one of the preceding claims, **characterised in that** the splint (4) consists of a thermoplastic synthetic material.

5. Orthosis according to one of the preceding claims, **characterised in that** the splint (4) is designed to partially enclose the wrist.

6. Orthosis according to one of the preceding claims, **characterised in that** the splint (4) is shaped to accommodate the thumb (2) in its natural resting position.

7. Orthosis according to one of the preceding claims, **characterised in that** the splint (4) surrounds the thumb joint and is designed as an abutment against the wrist and, when applied, lifts the thumb saddle joint out of the joint cavity.

## Revendications

1. Orthèse en matière plastique, comprenant une attelle (4) adaptée à l'anatomie, l'attelle (4) s'appliquant avec faculté de fixation amovible sur un poignet droit et du côté proximal de l'articulation carpo-métacarpienne du pouce de la main malade,
**caractérisée en ce que** l'attelle (4) se termine en dessous de l'articulation métacarpo-phalangienne du pouce, en étant menée en direction distale vers l'articulation métacarpo-phalangienne du pouce, du côté radial vers le côté ulnaire et du côté dorsal vers le côté palmaire en passant par l'éminence thénar (7), avec une spire gauche en forme de tire-bouchon.

2. Orthèse selon la revendication 1,
**caractérisée en ce qu'**une pelote d'appui proximal de l'articulation carpo-métacarpienne du pouce est incorporée dans l'attelle (4).

3. Orthèse selon la revendication 1 ou 2,
**caractérisée en ce que** l'orthèse peut être formée d'une matière plastique perforée.

4. Orthèse selon l'une des revendications précédentes,
**caractérisée en ce que** l'attelle (4) est constituée d'une matière thermoplastique.

5. Orthèse selon l'une des revendications précédentes,
**caractérisée en ce que** l'attelle (4) est réalisée de manière à entourer partiellement le poignet.

6. Orthèse selon l'une des revendications précédentes,
**caractérisée en ce que** l'attelle (4) est formée de manière à recevoir le pouce (2) dans sa position de repos naturelle.

7. Orthèse selon l'une des revendications précédentes,
**caractérisée en ce que** l'attelle (4) est réalisée de manière à entourer l'articulation du pouce et est conçue comme une contre-butée contre le poignet et, à l'état appliqué, fait effet de levier sur l'articulation carpo-métacarpienne du pouce pour la faire sortir de la cavité articulaire.
